(19) Europäisches Patentamt
European Patent Office
Office européen des brevets

(11) EP 1 023 891 B1

# (12) FASCICULE DE BREVET EUROPEEN

(45) Date de publication et mention de la délivrance du brevet:
**24.10.2001 Bulletin 2001/43**

(51) Int Cl.[7]: **A61K 7/135**

(21) Numéro de dépôt: **00400148.3**

(22) Date de dépôt: **20.01.2000**

(54) **Composition anhydre de décoloration des fibres kératiniques comprenant l'association de polymères amphiphiles anioniques et/ou non ioniques comportantau moins une chaîne grasse et de polymères substantifs cationiques ou amphotères**

Wasserfreie Zusammensetzung zum Bleichen von Keratinfasern, die anionische und/oder nichtionische amphiphile Polymere, die mindestens eine Fettkette aufweisen, in Kombination mit kationischen oder amphoteren substantiven Polymeren enthält

Anhydrous composition for bleaching keratin fibers containing anionic and/or non ionic amphiphilic polymers with at least one fatty chain and cationic or amphoteric polymers

(84) Etats contractants désignés:
**AT BE CH CY DE DK ES FI FR GB GR IE IT LI LU MC NL PT SE**

(30) Priorité: **29.01.1999 FR 9901054**

(43) Date de publication de la demande:
**02.08.2000 Bulletin 2000/31**

(73) Titulaire: **L'OREAL**
**75008 Paris (FR)**

(72) Inventeurs:
- **Legrand, Frédéric**
**92100 Boulogne-Billancourt (FR)**
- **Millequant, Jean**
**94100 Saint-Maur (FR)**

(74) Mandataire: **Casalonga, Axel**
**BUREAU D.A. CASALONGA - JOSSE**
**Morassistrasse 8**
**80469 München (DE)**

(56) Documents cités:
**EP-A- 0 827 738**
**EP-A- 0 882 444**
**WO-A-97/07776**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne des compositions anhydres pour la décoloration des fibres kératiniques, et en particulier des cheveux, comprenant l'association d'au moins un polymère amphiphile anionique comportant au moins une chaîne grasse et/ou d'au moins un polymère amphiphile non ionique comportant au moins une chaîne grasse et d'au moins un polymère substantif cationique ou amphotère, l'utilisation de ces compositions pour la préparation de compositions de décoloration prêtes-à-l'emploi, un procédé de décoloration de fibres kératiniques utilisant ces compositions et un kit d'emballage contenant une telle composition.

La décoloration des fibres kératiniques humaines, en particulier des cheveux, se fait par oxydation de la mélanine aboutissant à la solubilisation et élimination partielle ou totale de ce pigment.

Pour décolorer les cheveux, on utilise généralement des poudres décolorantes contenant un réactif peroxygéné tel que les persulfates, perborates ou percarbonates d'ammonium ou de métaux alcalins, que l'on associe au moment de l'emploi à une composition aqueuse de peroxyde d'hydrogène. Les sels peroxygénés et le peroxyde d'hydrogène étant relativement stables en milieu acide, il est nécessaire de les activer à pH basique pour obtenir une formation adéquate d'oxygène. Il est donc usuel d'ajouter aux poudres décolorantes des composés alcalins tels que les amines et les silicates alcalins.

Ce traitement chimique par des agents oxydants et alcalins est souvent très agressif et modifie la structure chimique de la kératine.

Ceci se traduit par de mauvaises propriétés cosmétiques des cheveux telles qu'un démêlage difficile, un toucher désagréable ou des cheveux rêches et ternes, mais surtout par une dégradation des fibres kératiniques.

Cette dégradation des fibres est particulièrement indésirable car elle détériore de manière irréversible les propriétés physico-chimiques des cheveux. Ceux-ci deviennent plus poreux et par conséquent plus difficile à sécher, ils présentent une plus grande sensibilité aux divers autres traitements capillaires tels qu'une coloration ou une permanente, et voient leurs propriétés mécaniques et leurs propriétés de surface modifiées défavorablement, ce qui se traduit par exemple par une diminution de la résistance à la rupture en traction ou une augmentation du coefficient de frottement.

Pour remédier à ces inconvénients, on a eu recours jusqu'ici à l'utilisation de polymères substantifs cationiques ou amphotères. Ces polymères, en se déposant sur les cheveux, améliorent leurs propriétés cosmétiques, c'est-à-dire les rendent plus doux, plus brillants et plus faciles à démêler mais ne permettent pas de limiter significativement la dégradation des fibres kératiniques.

La demanderesse a fait la découverte surprenante qu'il était possible de limiter significativement la dégradation des fibres kératiniques en combinant le ou les polymères substantifs cationiques ou amphotères utilisés habituellement avec un ou plusieurs polymères amphiphiles comportant au moins une chaîne grasse, également appelés *polymères associatifs*, anioniques et/ou non ioniques.

La présente invention a par conséquent pour objet une composition anhydre pour la décoloration de fibres kératiniques, en particulier humaines, comprenant, dans un milieu approprié pour la décoloration, au moins un agent alcalin, au moins un sel peroxygéné, et en outre l'association

- d'au moins un polymère amphiphile anionique et/ou non ionique comportant au moins une chaîne grasse, et
- d'au moins un polymère substantif cationique ou amphotère.

L'invention a également pour objet l'utilisation d'une telle composition anhydre pour la préparation d'une composition de décoloration prête-à-l'emploi.

Elle a en outre pour objet un procédé de décoloration des fibres kératiniques utilisant les compositions de décoloration anhydres ci-dessus, ainsi qu'un kit d'emballage contenant une telle composition.

D'autres objets apparaîtront à la lecture de la description et des exemples qui suivront.

Les polymères amphiphiles anioniques comportant au moins une chaîne grasse utilisés selon la présente invention en association avec les polymères substantifs cationiques ou amphotères sont des copolymères, réticulés ou non réticulés, comprenant

- des motifs *hydrophiles* dérivés d'un ou de plusieurs monomères à insaturation éthylénique portant une fonction d'acide carboxylique libre, et
- des motifs *hydrophobes* dérivés d'un ou de plusieurs monomères à insaturation éthylénique portant une chaîne latérale hydrophobe, et éventuellement
- des motifs de réticulation dérivés d'un ou de plusieurs monomères polyinsaturés.

Le ou les monomères à insaturation éthylénique portant une fonction d'acide carboxylique sont choisis parmi l'acide éthacrylique, l'acide méthacrylique et l'acide acrylique, de préférence parmi l'acide méthacrylique, l'acide acrylique et des mélanges de ceux-ci.

Le ou les monomères à insaturation éthylénique portant une chaîne latérale hydrophobe peuvent être (i) des esters d'acides carboxyliques insaturés et d'alcools gras, ou (ii) des éthers d'allyle et d'alcools gras.

(i) Les esters d'acides carboxyliques insaturés et d'alcools gras sont choisis par exemple parmi les éthacrylates, méthacrylates et/ou acrylates d'alkyle en $C_{10-30}$, de préférence en $C_{12-22}$.

Ils englobent par exemple l'acrylate de lauryle, l'acrylate de stéaryle, l'acrylate de décyle, l'acrylate d'isodécyle, l'acrylate de dodécyle ainsi que les méthacrylates correspondants, à savoir le méthacrylate de lauryle, le méthacrylate de stéaryle, le méthacrylate de décyle, le méthacrylate d'isodécyle et le méthacrylate de dodécyle.

(ii) Les éthers allyliques d'alcools gras formant les motifs hydrophobes des polymères amphiphiles anioniques de la présente invention correspondent à la formule

$$(I) \qquad CH_2{=}CR'CH_2\text{-}O\text{-}B_n\text{-}R$$

dans laquelle

R' représente un atome d'hydrogène ou un groupe méthyle,
B représente un groupe éthylèneoxy,
n est un nombre entier valant entre 0 et 100,
R représente un groupe hydrocarboné choisi parmi les restes alkyle, arylalkyle, aryle, alkylaryle ou cycloalkyle comportant de 8 à 30 atomes de carbone, de préférence de 10 à 24 atomes de carbone, et plus particulièrement de 12 à 18 atomes de carbone.

Un motif de formule (I) préféré selon la présente invention est un motif dans lequel R' désigne un atome d'hydrogène, n est égal à 10 et R représente un radical stéaryle ($C_{18}$).

Ledit monomère réticulant est un composé comportant au moins deux doubles liaisons polymérisables non conjuguées l'une avec l'autre. On peut citer à titre d'exemple le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.

Des polymères amphiphiles anioniques du type décrit ci-dessus sont décrits par exemple dans les brevets US-3 915 921 et US-4 509 949 (copolymères d'acide (m)éthacrylique et de (m)éthacrylates d'alkyle en $C_{10-30}$), ou dans le brevet EP-0 216 479 B2 (copolymères d'acide (m)éthacrylique et d'éthers allyliques d'alcools gras).

On peut citer à titre d'exemples de polymères préférés :

- les polymères réticulés d'acide acrylique et d'acrylate d'alkyle en $C_{10-30}$, tels que les polymères commercialisés sous les dénominations PEMULEN TR1, PEMULEN TR2 et CARBOPOL 1382 par la société GOODRICH,
- les polymères réticulés d'acide acrylique et de méthacrylate d'alkyle en $C_{10-30}$, tels que le CARBOPOL ETD 2020 commercialisé par la société GOODRICH,
- le terpolymère acide méthacrylique/acrylate d'éthyle/méthacrylate de stéaryle oxyéthyléné (55/35/10),
- le terpolymère acide (méth)acrylique/acrylate d'éthyle/méthacrylate de béhényle oxyéthyléné (25 OE), et
- le terpolymère acide méthacrylique/acrylate d'éthyle/stéareth-10 allyléther réticulé.

Les polymères amphiphiles non ioniques comportant au moins une chaîne grasse utilisables selon la présente invention englobent par exemple

- les celluloses ou hydroxyalkylcelluloses modifiées par des groupements comportant au moins une chaîne grasse telle qu'un groupe alkyle, arylalkyle ou alkylaryle contenant un groupe alkyle de préférence en $C_{8-22}$ comme les produits NATROSOL PLUS GRADE 330 CS de la société AQUALON, BERMOCOLL EHM 100 de la société BEROL NOBEL, ou POLYSURF 67 de la société HERCULES, ou modifiées par des groupes alkylphénol polyalcoxylés comme le produit AMERCELL POLYMER HM-1500 de la société AMERCHOL,
- les hydroxypropylguars modifiés par des groupements comportant au moins une chaîne grasse en $C_{8-22}$ comme les produits ESAFLOR HM 22 (chaîne alkyle en $C_{22}$) de la société LAMBERTI, MIRACARE XC95-3 (chaîne alkyle en $C_{14}$) et RE205-1 (chaîne alkyle en $C_{20}$) de la société RHONE POULENC,
- les polyuréthannes comportant au moins une chaîne grasse de type alkyle ou alcényle en $C_{8-30}$ comme le produit SER-AD FX 1100 de la société SERVODELBEN , ou
- le copolymère SMDI (Saturated Methylene Diphenyl Diisocyanate) polyéthylèneglycol(s) avec terminaison décyle,

- le copolymère SMDI (Saturated Methylene Diphenyl Diisocyanate) polyéthylène glycol(s) avec terminaison alkyle (méthyle/$C_{18}$), associé à une matrice maltodextrine, et
- le diuréthane HMDI (Hexa Methylene Di Isocyanate) d'alcools en $C_{10-18}$ oxyéthylénés (66 OE) et oxypropylénés (14 OP) commercialisé sous la dénomination ELFACOS T 212 par la société AKZO.
- les copolymères de vinylpyrrolidone et de monomères hydrophobes à chaîne grasse comme les produits ANTARON V216 ou GANEX V216 (poly(vinylpyrrolidone/hexadécène)), ANTARON V220 ou GANEX V220 (poly(vinylpyrrolidone/eicosène)) de la société I.S.P. ;
- les copolymères de (méth)acrylates d'alkyle en $C_{1-6}$ et de monomères amphiphiles comportant au moins une chaîne grasse ;
- les copolymères de (méth)acrylates hydrophiles et de monomères hydrophobes comportant au moins une chaîne grasse, par exemple un poly(méthacrylate de polyéthylèneglycol/méthacrylate de lauryle).

On préfère en particulier les polyuréthannes comportant au moins une chaîne grasse de type alkyle en $C_{10-20}$ et les hydroxyéthylcelluloses modifiées par des groupements comportant au moins un groupe alkyle en $C_{8-22}$.

Les compositions anhydres contiennent le ou les polymère(s) amphiphile(s) anionique(s) et/ou non ionique(s) comportant au moins une chaîne grasse de préférence à raison de 0,03 à 30 % en poids, et en particulier à raison de 0,3 à 15 % en poids.

Dans les compositions de décoloration de la présente invention on associe le ou les polymère(s) amphiphile(s) anionique(s) et/ou non ionique(s) comportant au moins une chaîne grasse décrits ci-dessus à au moins un polymère substantif cationique ou amphotère.

Dans le domaine cosmétique, on entend par polymère *substantif* un polymère capable, grâce à sa forte affinité pour un support tel que les cheveux, de former un dépôt sur celui-ci. Le caractère substantif est classiquement évalué au moyen du test décrit par Richard J. Crawford, *Journal of the Society of Cosmetic Chemists*, 1980, 31(5), pages 273 - 278.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans les demandes de brevet EP-A-337354 et EP-A-557203 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :

(1) les homopolymères ou copolymères dérivés d'esters ou d'amides acryliques ou méthacryliques et comportant au moins un des motifs de formules (II), (III), (IV) ou (V) suivantes:

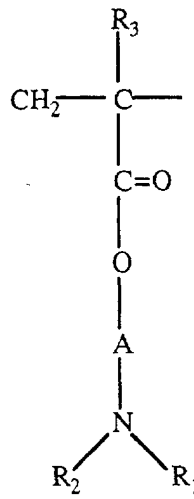

(II) ,

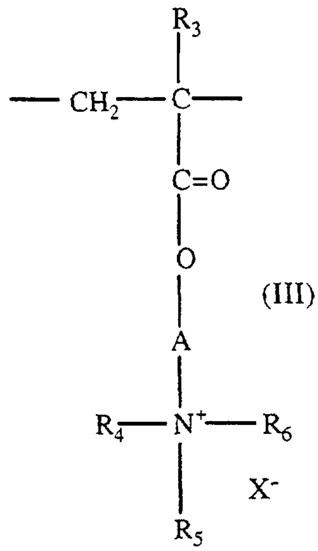

(III)

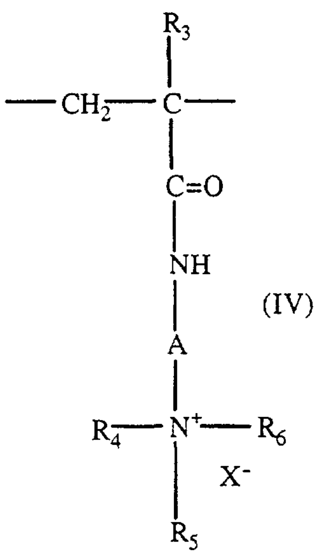

(IV)

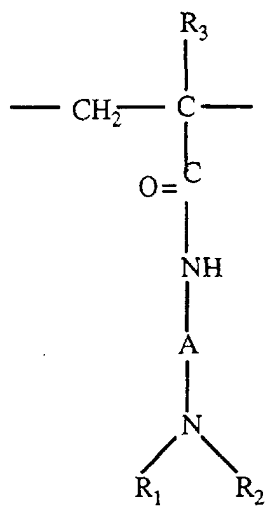

(V)

dans lesquelles:

$R_3$, identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$, identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques.

Ainsi, parmi ces polymères de la famille (1), on peut citer :

- les copolymères d'acrylamide et de diméthyl-amino-éthyl méthacrylate quaternisé au sulfate de diméthyle ou avec un halogénure de diméthyle,
- les copolymères d'acrylamide et de chlorure de méthacryloyloxyéthyltriméthylammonium décrits par exemple dans la demande de brevet EP-A-080976,
- le copolymère d'acrylamide et de méthosulfate de méthacryloyloxy-éthyltriméthyl-ammonium,
- les copolymères vinylpyrrolidone/acrylate ou méthacrylate de dialkylaminoalkyle quaternisés ou non. Ces polymères sont décrits en détail dans les brevets français 2.077.143 et 2.393.573,
- les terpolymères méthacrylate de diméthylaminoéthyle/ vinylcaprolactame/vinylpyrrolidone,
- les copolymère vinylpyrrolidone/méthacrylamidopropyldimethylamine,
- et les copolymères vinylpyrrolidone/méthacrylamide de diméthylaminopropyle quaternisés.

(2) Les dérivés d'éthers de cellulose comportant des groupements ammonium quaternaire décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyltriméthylammonium, de méthacrylmidopropyltriméthylammonium, de diméthyldiallylammonium.

(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.

(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bisinsaturé, une bis-halohydrine, un bis-azétidinium, une bishaloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalogénhydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .

(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylaminohydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire du polyalkylène polyamine à l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

(9) Les cyclopolymères d'alkyl diallyl amine ou de dialkyldiallylammonium tels que les homopolymères ou copolymères comportant comme constituant principal de la chaîne des motifs répondant aux formules (VI) ou (VII) :

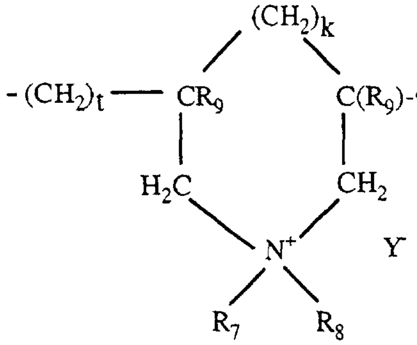

(VI)

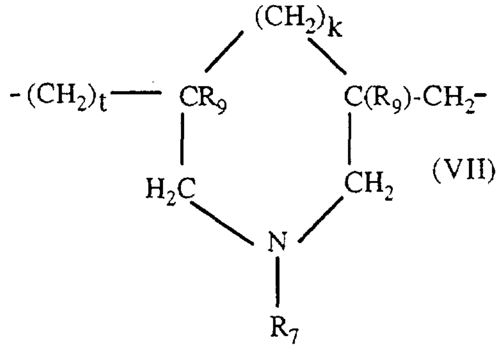

(VII)

formules dans lesquelles k et t sont égaux à 0 ou 1, la somme k + t étant égale à 1 ; $R_9$ désigne un atome d'hydrogène ou un radical méthyle ; $R_7$ et $R_8$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

(10) Le polymère de diammonium quaternaire contenant des motifs récurrents répondant à la formule :

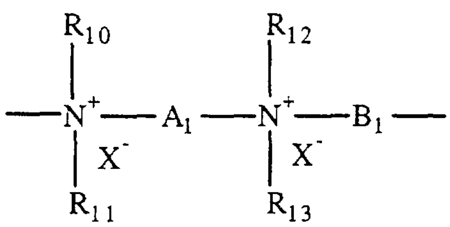

(VIII)

dans laquelle $R_{10}$, $R_{11}$, $R_{12}$ et $R_{13}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{10}$, $R_{11}$, $R_{12}$ et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O-$R_{14}$-D ou -CO-NH-$R_{14}$-D où $R_{14}$ est un alkylène et D un groupement ammonium quaternaire ;

$A_1$ et $B_1$ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
$X^-$ désigne un anion dérivé d'un acide minéral ou organique;
$A_1$, $R_{10}$ et $R_{12}$ peuvent former avec les deux atomes d'azote auxquels ils sont rattachés un cycle pipérazinique ; en outre si $A_1$ désigne un radical alkylène ou hydroxyalkylène linéaire ou ramifié, saturé ou insaturé, $B_1$ peut également désigner un groupement

$$-(CH_2)_n-CO-D-OC-(CH_2)_n-$$

dans lequel D désigne :

a) un reste de glycol de formule :
-O-Z-O-, où Z désigne un radical hydrocarboné linéaire ou ramifié ou un groupement répondant à l'une des formules suivantes :

$$-(CH_2-CH_2-O)_x-CH_2-CH_2-$$

$$-[CH_2-CH(CH_3)-O]_y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH- ;

De préférence, $X^-$ est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (IX) suivante:

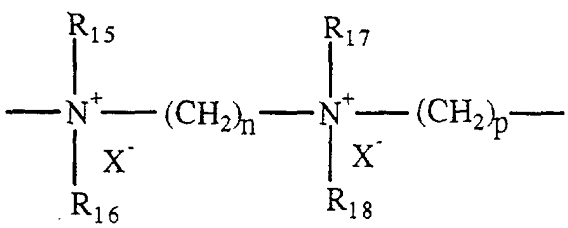

(IX)

dans laquelle $R_{15}$, $R_{16}$, $R_{17}$ et $R_{18}$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

(11) Les polymères de polyammonium quaternaires constitués de motifs de formule (X):

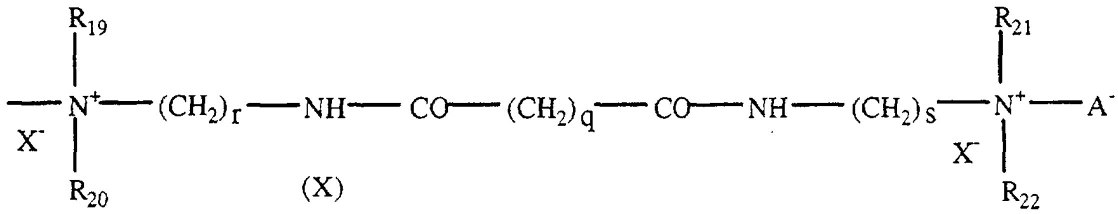

(X)

formule dans laquelle :

$R_{19}$, $R_{20}$, $R_{21}$ et $R_{22}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$, où p est égal à 0 ou à un nombre entier compris entre 1 et 6, sous réserve que $R_{19}$, $R_{20}$, $R_{21}$ et $R_{22}$ ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence $-CH_2-CH_2-O-CH_2-CH_2-$.

De tels composés sont notamment décrits dans la demande de brevet EP-A-122 324.

(12) Les polymères quaternaires de vinylpyrrolidone et de vinylimidazole.

(13) Les polyamines comme le produit référencé sous le nom de POLYETHYLENEGLYCOL (15) TALLOW POLYAMINE dans le dictionnaire CTFA.

(14) Les polymères réticulés de sels de méthacryloyl-oxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homopolymérisation ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyltriméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de SALCARE® SC 95 et SALCARE® SC 96 par la Société ALLIED COLLOIDS. On peut également utiliser le méthosulfate du copolymère de méthacryloyloxyéthyltriméthylammonium et de méthacryloyloxyéthyldiméthylacétylammonium, commercialisé sous la dénomination PLEX 7525L par la société ROHM GmbH (dénomination CTFA : Polyquaternium-35).

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères filmogènes amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les méthacrylates et -acrylates de dialkylaminoalkyle, les dialkylaminoalkylméthacrylamide et -acrylamide. De tels composés sont décrits dans le brevet américain n°3 836 537. On peut également citer le copolymère acrylate de sodium/chlorure d'acrylamidopropyltriméthylammonium.

Le composé vinylique peut être également un sel de dialkyldiallylammonium tel que le chlorure de diéthyldiallylammonium.

(2) Les polymères comportant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou de diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.

Les comonomères basiques préférés sont des méthacrylates d'aminoéthyle, de butylaminoéthyle, de N,N'-diméthylaminoéthyle, de N-tertio-butylaminoéthyle.

On utilise particulièrement les copolymères dont la dénomination CTFA (4ème édition, 1991) est Octylacrylamide/acrylates/butylaminoethylmethacrylate copolymer.

(3) Les polyaminoamides réticulés et alcoylés partiellement ou totalement dérivant de polyaminoamides de formule générale :

$$-\!\!\left[CO - R_{23} - CO - Z\right]\!\!- \qquad (XI)$$

dans laquelle $R_{23}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

$$-NH\left[(CH_2)_x - NH\right]_p- \qquad (XII)$$

où x=2 et p=2 ou 3, ou bien x=3 et p=2, ce radical dérivant de la diéthylène triamine, de la triéthylène tétraamine ou de la dipropylène triamine;
b) dans les proportions de 0 à 40 moles % le radical (XII) ci-dessus, dans lequel x=2 et p=1 et qui dérive de l'éthylènediamine, ou le radical dérivant de la pipérazine :

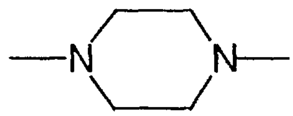

c) dans les proportions de 0 à 20 moles % le radical

$$-NH-(CH_2)_6-NH-$$

dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanesultones utilisées dans l'alcoylation sont de préférence la propanesultone ou la butanesultone, les sels des agents d'alcoylation sont de préférence les sels de sodium ou de potassium.

(4) Les polymères comportant des motifs zwitterioniques de formule :

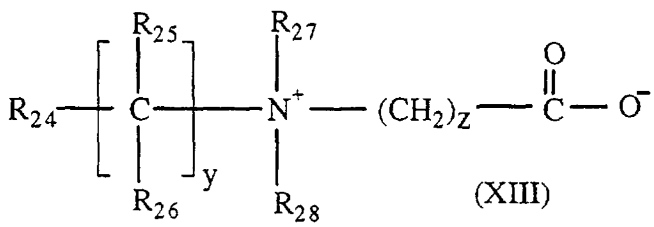

(XIII)

dans laquelle $R_{24}$ désigne un groupement insaturé polymérisable tel qu'un groupement acrylate, méthacrylate, acrylamide ou méthacrylamide, y et z représentent un nombre entier de 1 3, $R_{25}$ et $R_{26}$ représentent un atome d'hydrogène, méthyle, éthyle ou propyle, $R_{27}$ et $R_{28}$ représentent un atome d'hydrogène ou un radical alkyle de telle façon que la somme des atomes de carbone dans $R_{27}$ et $R_{28}$ ne dépasse pas 10.

Les polymères comprenant de telles unités peuvent également comporter des motifs dérivés de monomères non zwitterioniques tels que l'acrylate ou le méthacrylate de diméthyl- ou diéthylaminoéthyle ou des acrylates ou méthacrylates d'alkyle, des acrylamides ou méthacrylamides ou l'acétate de vinyle.

A titre d'exemple, on peut citer le copolymère de méthacrylate de méthyle/diméthylcarboxy-méthyl-ammonioéthyl-méthacrylate de méthyle.

(5) les polymères dérivés du chitosane comportant des motifs monomères répondant aux formules (XIV), (XV), (XVI) suivantes :

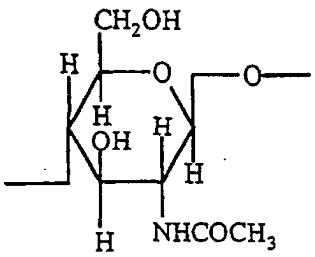

(XIV)

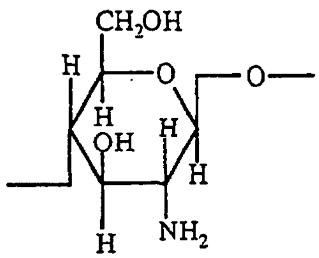

(XV)

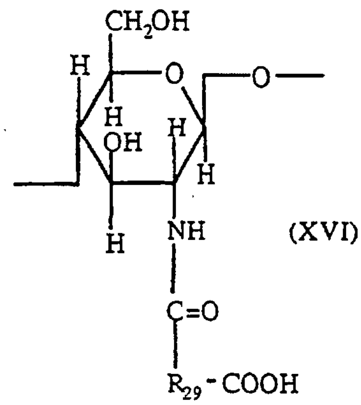

(XVI)

le motif XIV étant présent dans des proportions comprises entre 0 et 30%, le motif XV dans des proportions comprises entre 5 et 50% et le motif XVI dans des proportions comprises entre 30 et 90%, étant entendu que dans ce motif F, $R_{29}$ représente un radical de formule :

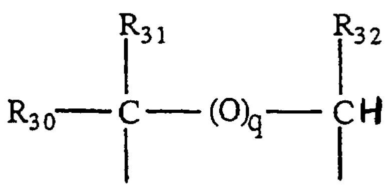

(XVII)

dans laquelle

si q=0, $R_{30}$, $R_{31}$ et $R_{32}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux $R_{30}$, $R_{31}$ et $R_{32}$ étant dans ce cas un atome d'hydrogène ;
ou si q=1, $R_{30}$, $R_{31}$ et $R_{32}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutylchitosane.

(7) Les polymères répondant à la formule générale (XVIII) tels que ceux décrits par exemple dans le brevet français 1 400 366 :

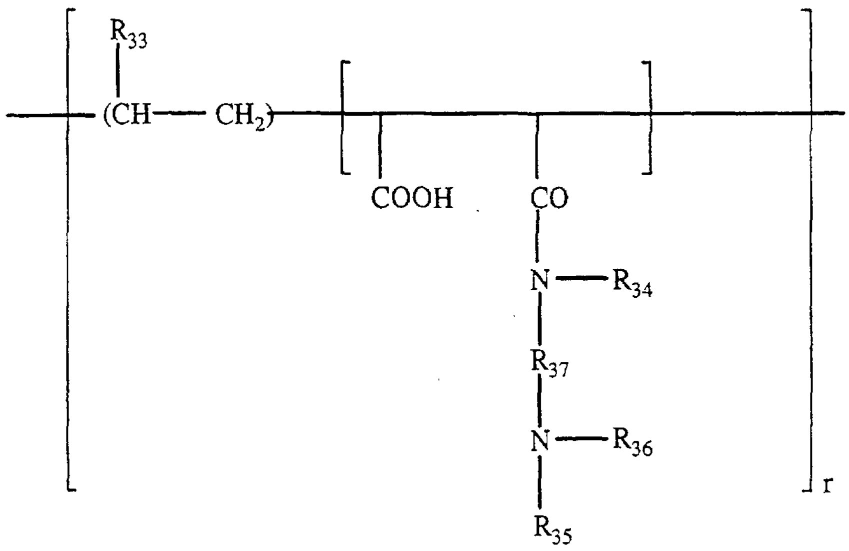

(XVIII)

dans laquelle $R_{33}$ représente un atome d'hydrogène, un radical $CH_3O$, $CH_3CH_2O$, phényle, $R_{34}$ désigne l'hydrogène ou un radical alkyle inférieur tel que méthyle, éthyle, $R_{35}$ désigne l'hydrogène ou un radical alkyle inférieur tel que méthyle, éthyle, $R_{36}$ désigne un radical alkyle inférieur tel que méthyle, éthyle ou un radical répondant à la formule :

- $R_{37}$-$N(R_{35})_2$, $R_{37}$ représentant un groupement $-CH_2-CH_2-$,
- $CH_2-CH_2-CH_2-$, $-CH_2-CH(CH_3)-$, et $R_{35}$ ayant les significations mentionnées ci-dessus, ainsi que les homologues supérieurs de ces radicaux et contenant jusqu'à 6 atomes de carbone.

(8) Des polymères amphotères du type -D-X-D-X- choisis parmi:

a) les polymères obtenus par action de l'acide chloracétique ou le chloracétate de sodium sur les composés comportant au moins un motif de formule :

$$-D-X-D-X-D- \qquad (XIX)$$

où D désigne un radical

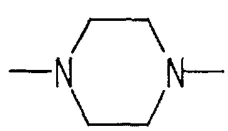

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en'outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;

b) les polymères de formule :

$$-D-X-D-X- \qquad (XX)$$

où D désigne un radical

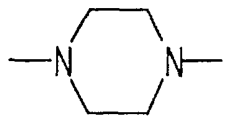

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)-vinyléther/anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères substantifs cationiques ou amphotères peuvent également porter des groupements hydrophobes.

Ils sont choisis par exemple parmi les dérivés de cellulose à groupements ammonium quaternaire et les polyacrylates à groupements latéraux aminés, comme par exemple :

- les celluloses à groupements ammonium quaternaires, modifiées par des groupements comportant au moins une chaîne grasse, choisis parmi les groupes alkyle, arylalkyle, alkylaryle comportant au moins 8 atomes de carbone, ou des mélanges de ceux-ci,
- les hydroxyéthylcelluloses à groupements ammonium quaternaire modifiées par des groupements comportant au moins une chaîne grasse, choisis parmi les groupes alkyle, arylalkyle, alkylaryle comportant au moins 8 atomes de carbone, ou des mélanges de ceux-ci.
- les polyacrylates à groupements latéraux aminés, quaternisés ou non, possédant des groupements hydrophobes.

Les radicaux alkyle portés par les celluloses ou hydroxyéthylcelluloses à groupements ammonium quaternaire ci-dessus comportent de préférence de 8 à 30 atomes de carbone.

Les radicaux aryle désignent de préférence les groupements phényle, benzyle, naphtyle ou anthryle.

Parmi les polymères substantifs cationiques ou amphotères utilisables selon l'invention, on préfère notamment :

- l'homopolymère de chlorure de diméthyldiallylammonium vendu sous la dénomination MERQUAT 100 DRY par la société MERCK ;
- les copolymères de chlorure de diméthyldiallylammonium et d'acrylamide vendus sous la dénomination MERQUAT 2200 par la société CALGON;
- les polymères de type poly(ammonium quaternaire) préparés et décrits dans le brevet français 2 270 846, constitués de motifs récurrents répondant à la formule (XXI) suivante :

(XXI) 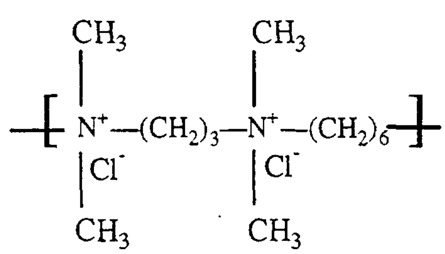

et notamment ceux dont la masse molaire moyenne en poids, déterminée par chromatographie par perméation

de gel, est comprise entre 9500 et 9900 ;

- les polymères de type poly(ammonium quaternaire) préparés et décrits dans le brevet français 2 270 846, constitués de motifs récurrents répondant à la formule (XXII) suivante :

(XXII) 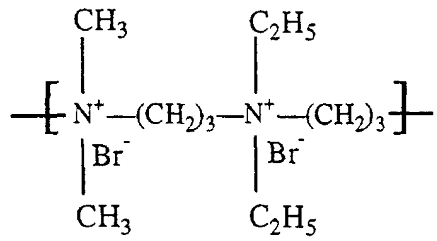

et notamment ceux dont la masse molaire moyenne en poids, déterminée par chromatographie par perméation de gel, est d'environ 1200 ;

- les polymères de type poly(ammonium quaternaire) décrits dans les brevets US 4 390 689, 4 702 906, 4 719 282 et constitués de motifs récurrents répondant à la formule (XXIII) suivante :

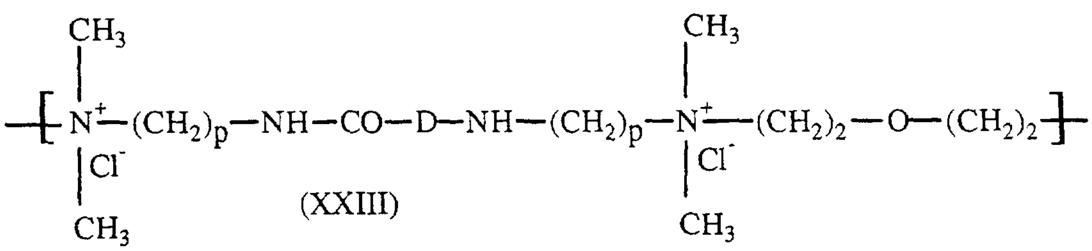

(XXIII)

dans laquelle

p est un nombre entier valant entre 1 et 6,
D représente une simple liaison ou un groupe $-(CH_2)_r-CO-$ où r vaut 4 ou 7 ,
et notamment ceux dont la masse molaire moyenne en poids est inférieure à 100 000, de préférence inférieure ou égale à 50 000;

- les copolymères amphotères suivants :
- le copolymère chlorure de diallyldiméthylammonium/acide acrylique (80/20) commercialisé sous la dénomination MERQUAT 280 DRY par la société CALGON (dénomination CTFA : Polyquaternium-22);
- le copolymère chlorure de diméthyldiallylammonium/acide acrylique (95/5) vendu sous la dénomination MERQUAT 295 DRY par la société CALGON (dénomination CTFA : Polyquaternium-22);
- le copolymère de chlorure de méthacrylamidopropyltrimonium, d'acide acrylique et d'acrylate de méthyle, commercialisé sou la dénomination MERQUAT 2001 par la société CALGON (dénomination CTFA : Polyquaternium-47) ; et
- le terpolymère acrylamide/chlorure de diméthyldiallylammonium/acide acrylique commercialisé sous la dénomination MERQUAT PLUS 3330 DRY par la société CALGON (dénomination CTFA : Polyquaternium-39) .

Dans la liste des polymères substantifs ci-dessus, on préfère tout particulièrement les copolymères amphotères Polyquaternium-22, Polyquaternium-39 et Polyquaternium-47 (dénominations CTFA).

Les compositions de décoloration anhydres contiennent le polymère substantif cationique ou amphotère de préférence à raison 0,03 à 30 % en poids, et en particulier à raison de 0,3 à 15 % en poids, rapporté à la composition anhydre.

Le rapport en poids du ou des polymère(s) amphiphile(s) anionique(s) et/ou non ionique(s) au polymère substantif cationique ou amphotère est généralement compris entre 10/1 et 1/10 et de préférence entre 5/1 et 1/5.

Comme indiqué ci-dessus, la composition décolorante anhydre contient au moins un agent alcalin et au moins un sel peroxygéné.

Ledit agent alcalin est choisi parmi les sels d'ammonium tels que le chlorure, le sulfate, le phosphate ou le

nitrate d'ammonium, les silicates, phosphates ou carbonates de métaux alcalins ou alcalino-terreux, en particulier les métasilicates de métaux alcalins.

Les sels peroxygénés sont choisis parmi les persulfates, les percarbonates et les perborates d'ammonium ou de métaux alcalins.

On utilise de préférence les persulfates et parmi ceux-ci principalement les persulfates de sodium et de potassium.

Les compositions de l'invention contiennent de préférence entre 20 et 70 % en poids, et en particulier entre 30 et 60 % en poids de sel peroxygéné, par rapport au poids total de la composition anhydre.

Les compositions de décoloration selon la présente invention peuvent également contenir toutes sortes d'adjuvants utilisés habituellement dans les compositions de décoloration susceptibles de faciliter la manipulation et l'application, d'améliorer la conservation ou l'efficacité des compositions et d'améliorer les propriétés cosmétiques des cheveux traités.

Ces adjuvants sont par exemple des agents de contrôle du dégagement d'oxygène tels que le carbonate de magnésium et la magnésie, des polymères épaississants ou gélifiants solubles dans l'eau tels que les celluloses et leurs dérivés comme l'hydroxyéthylcellulose, l'hydroxypropylcellulose et l'hydroxypropylméthylcellulose, l'amidon et les dérivés d'amidon, l'hydroxypropylguar et la gomme de guar, les alginates, les polysaccharides, la polyvinylpyrrolidone, la carboxyméthylcellulose, la gomme de xanthane, la gomme arabique, la gomme ghatti, la gomme adragante, les polyacrylamides et les poly(acide acrylique), des agents tensio-actifs anioniques, non ioniques, cationiques, amphotères ou zwitterioniques et leur mélanges, des huiles minérales ou végétales, des cires, des adjuvants de granulation, des liants, des charges minérales telles que la silice et l'argile, des opacifiants tels que l'oxyde de titane, des colorants, des séquestrants et des parfums.

Bien entendu, l'homme de métier veillera à choisir ce (ou ces) éventuel(s) composé(s) supplémentaire(s) et sa (ou leur) quantité de manière à ce que les propriétés avantageuses attachées intrinsèquement à la composition de décoloration conforme à l'invention ne soient pas, ou substantiellement pas, altérées par la (ou les) adjonctions envisagées.

Les compositions de l'invention comprennent de préférence au moins un agent tensio-actif.

Les agents tensio-actifs convenant à la mise en oeuvre de la présente invention sont notamment les suivants :

(i) <u>Tensio-actif(s) anionique(s)</u> :

A titre d'exemple de tensio-actifs anioniques utilisables, seuls ou mélanges, dans le cadre de la présente invention, on peut citer notamment les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkyl($C_6$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl($C_6$-$C_{24}$) polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) <u>Tensio-actif(s) non ionique(s)</u> :

Les agents tensio-actifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi les alcools, les alpha-diols, les alkylphénols ou les acides gras polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant

en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10-14}$) amines ou les oxydes de N-acylaminopropylmorpholine. On notera que les alkylpolyglycosides constituent des tensio-actifs non-ioniques rentrant particulièrement bien dans le cadre de la présente invention.

(iii) Tensio-actif(s) amphotère(s) ou zwitterionique(s) :

Les agents tensio-actifs amphotères ou zwitterioniques, dont la nature ne revêt pas dans le cadre de la présente invention de caractère critique, peuvent être notamment des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_{38}\text{-CONHCH}_2\text{CH}_2\text{-N}(R_{39})(R_{40})(\text{CH}_2\text{COO-})$$

dans laquelle : $R_{38}$ désigne un radical alkyle d'un acide $R_{38}$-COOH présent dans l'huile de coprah hydrolysée, un radical heptyle, nonyle ou undécyle, $R_{39}$ désigne un groupement bêta-hydroxyéthyle et $R_{40}$ un groupement carboxyméthyle ;

et

$$R_{38}'\text{-CONHCH}_2\text{CH}_2\text{-N(B)(C)}$$

dans laquelle :

B représente $-CH_2CH_2OX'$,
C représente $-(CH_2)_z-Y'$, avec z = 1 ou 2,
X' désigne le groupement $-CH_2CH_2-COOH$ ou un atome d'hydrogène
Y' désigne -COOH ou le radical $-CH_2-CHOH-SO_3H$
$R_{38}'$ désigne un radical alkyle d'un acide R-COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5$^{ème}$ édition, 1993, sous les dénominations Disodium Coco-ampho-diacetate, Disodium Lauroampho-diacetate, Disodium Capryl-ampho-diacetate, Disodium Capryloampho-diacetate, Disodium Coco-amphodipropionate, Disodium Lauroampho-dipropionate, Disodium Caprylampho-dipropionate, Disodium Capryloampho-dipropionate, Lauroampho-dipropionic acid, Coco-ampho-dipropionic acid.

(iv) Tensio-actifs cationiques :

Parmi les tensio-actifs cationiques on peut citer en particulier : les sels d'amines grasses primaires, secondaires ou tertiaires, éventuellement polyoxyalkylénées, les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,1 à 30% du poids total de la composition.

La composition décolorante anhydre peut se présenter sous forme d'une poudre donnant naissance, après mélange avec de l'eau oxygénée, à un cataplasme. Elle peut également se présenter sous forme d'une crème anhydre décolorante contenant des agents pulvérulents en suspension ou en dispersion dans un support organique, telle que les crèmes décrites dans les brevets US 4 170 637, DE 3 814 356, DE 3 844 956, EP 0 778 020 et DE 1 972 3538 .

Selon la présente invention, la composition décolorante anhydre se présente de préférence sous forme d'une poudre de particules enrobées, non enrobées ou granulées.

La présente invention à également pour objet un procédé de décoloration des fibres kératiniques, en particulier des cheveux humains.

Ce procédé comprend les étapes consistant

- à mélanger, immédiatement avant emploi, la composition décolorante anhydre contenant au moins un agent alcalin, au moins un sel peroxygéné, et l'association d'au moins un polymère amphiphile anionique et/ou non ionique comportant au moins une chaîne grasse et d'au moins un polymère substantif cationique ou amphotère, avec une composition aqueuse de peroxyde d'hydrogène,
- à appliquer le mélange sur la zone des fibres kératiniques à décolorer,
- à laisser reposer pendant un temps suffisant pour obtenir la décoloration recherchée, temps généralement compris entre 10 minutes et une heure, de préférence entre 10 et 45 minutes, et
- à éliminer le mélange de décoloration par rinçage à l'eau, suivi d'un lavage avec un shampooing, puis d'un séchage.

L'invention a en outre pour objet l'utilisation d'une composition décolorante anhydre telle que décrite ci-dessus pour la préparation d'une composition décolorante prête-à-l'emploi. Pour cela la composition anhydre est mélangée avec environ 0,5 à 10 équivalents en poids d'une composition aqueuse de peroxyde d'hydrogène, par exemple une solution, une émulsion ou un gel, ayant une concentration pondérale comprise entre 2 et 12 %. Ce mélange doit se faire immédiatement avant l'application du produit sur les cheveux.

Le pH de la composition décolorante prête-à-l'emploi est compris de préférence entre 7 et 12 et plus préférentiellement encore entre 8,5 et 11,5.

Un autre objet de l'invention est un dispositif d'emballage en plusieurs parties, également appelé "kit" d'emballage, comprenant au moins deux compartiments dont l'un contient une composition décolorante anhydre telle que décrite ci-dessus, et l'autre une composition aqueuse de peroxyde d'hydrogène.

Les exemples, donnés ci-après à titre purement illustratif et non limitatif, permettront de mieux comprendre l'invention.

**Exemples 1 à 3**

On a préparé les trois compositions de décoloration sous forme pulvérulente, **A**, **B** et **C** suivantes :

| | quantités (en % en poids) | | |
|---|---|---|---|
| | composition A | composition B | composition C (invention) |
| persulfate de potassium | 35 | 35 | 35 |
| persulfate de sodium | 30 | 30 | 30 |
| métasilicate de sodium | 14 | 14 | 14 |
| chlorure d'ammonium | 5 | 5 | 5 |
| acide éthylènediamine tétraacétique | 1 | 1 | 1 |
| dioctylsulfosuccinate de Na/ benzoate de sodium | 1 | 1 | 1 |
| stéarate de calcium | 1 | 1 | 1 |
| silice | 9 | 8 | 6,5 |
| Polyquaternium-22* | 0 | 1 | 1 |
| copolymère réticulé (acide acrylique/acrylate d'alkyle en $C_{10-30}$)** | 0 | 0 | 1,5 |
| gomme de guar *** | 2 | 2 | 2 |
| hydroxyéthylcellulose | 2 | 2 | 2 |

* Merquat 280 dry vendu par la société CALGON.

** Carbopol 1382 commercialisé par la société GOODRICH

*** vendue sous la dénomination GUARGEL D/15 par la Société Française des Colloïdes

On a préparé également la composition de peroxyde d'hydrogène D suivante

| | composition D (quantité en % en poids) |
|---|---|
| alcool cétéarylique/ceteareth-30 | 2,85 |
| stabilisants | 0,06 |
| séquestrant | 0,15 |
| peroxyde d'hydrogène | 9 |
| acide phosphorique | qsp pH 2 |
| eau distillée | qsp 100 |

On a mélangé 8 g de chacune des compositions de décoloration A, B et C avec 16 g de la composition D de peroxyde d'hydrogène.

On a réalisé avec chacune des compositions prêtes-à-l'emploi AD, BD et CD 3 cycles de décoloration successifs sur des mèches de cheveux châtains de 3 g suivant le protocole suivant :

On a immergé dans chacune des compositions prêtes-à-l'emploi, après déclenchement du chronomètre, une mèche de cheveux que l'on a étalé ensuite en éventail sur une plaque thermostatée à 31 °C. On a recouvert la mèche en éventail de la moitié du mélange et on a laissé poser pendant 20 minutes. Au bout de 20 minutes, on a retourné rapidement la mèche, on l'a recouverte avec le reste du mélange et on a laissé poser 20 minutes supplémentaires. A

la fin du temps de pose, on a rincé abondamment la mèche à l'eau jusqu'à ce que celle-ci reste limpide. On a effectué un shampooing neutralisant afin d'éliminer d'éventuelles traces d'agent oxydant. On a démêlé la mèche au peigne, on l'a séché pendant 1 heure au séchoir électrique à 60 °C en la protégeant avec du papier absorbant. On a mouillé à nouveau la mèche et on l'a lavée de nouveau avec un shampooing classique. On a démêlé, puis on sèche à nouveau pendant 1 heure au séchoir électrique à 60 °C.

Après décoloration, chaque mèche de 3 g a été divisée en 5 mèches de 0,6 g, chacune de ces mèches étant soumise à l'essai de solubilité alcaline décrit ci-dessous.

On a déposé chacune des 5 mèches dans un cristallisoir que l'on a mis à l'étuve à 60 °C pendant 30 minutes ; on a pesé les cristallisoirs contenant les mèches après passage à l'étuve (**$P_0$**). On a sorti les mèches des cristallisoirs et on a pesé ces mêmes cristallisoirs vides (**$P_1$**). Ensuite, on a replacé les mèches dans les cristallisoirs que l'on a placés dans un dessiccateur pendant 24 heures.

Dans des fioles erlenmeyer de 50 ml on a introduit 40 ml d'une solution NaOH (0,1 N) et on a mis les fioles au bain-marie thermostaté à 65 °C. Lorsque la température a été stabilisée, on a immergé les mèches de cheveux à tester pendant 30 minutes dans la solution alcaline en remuant 2 à 3 fois avec précaution. On a sorti les mèches de cheveux, puis on a procédé à une série de trois lavages, chaque lavage consistant à immerger la mèche pendant 15 minutes dans 100 ml d'eau déminéralisée en agitant délicatement de temps en temps. On a laissé ensuite égoutter les mèches ainsi rincées et on les a déposées dans les cristallisoirs préalablement tarés (**$P_1$**). L'ensemble cristallisoir + mèche a été mis à l'étuve et séché pendant 24 heures à 105 °C. Après refroidissement pendant 45 minutes dans un dessiccateur, on a pesé à nouveau les cristallisoirs contenant les mèches (**$P_2$**).

La solubilité alcaline (SA) mesurant la perte de poids de la mèche, exprimée en %, a été calculée de la manière suivante :

$$SA = (P_0 - P_2) / (P_0 - P_1 - a) \times 100$$

où (**$P_0$-$P_2$**) représente la perte en poids de la mèche pendant l'essai et (**$P_0$-$P_1$-a**) représente le poids initial de la mèche, **a** correspondant au poids de l'attache des mèches (0,07 g).

La mesure de la solubilité alcaline des cheveux permet de rendre compte de la dégradation des fibres kératiniques résultant de leur décoloration. En effet, l'oxydation des ponts disulfure de la kératine en groupements acide sulfonique au cours de la décoloration augmente la solubilité du cheveu traité dans une solution alcaline. Plus la solubilité alcaline est élevée, plus l'état de la fibre est dégradé.

Les résultats de ces essais de solubilité alcaline résumés dans le tableau ci-dessous sont les valeurs moyennes calculées à partir des résultats des 5 mèches individuelles.

| | solubilité alcaline (en %) | |
|---|---|---|
| | moyenne | écart-type |
| composition AD | 54,5 | 3,1 |
| composition BD | 54,6 | 2,1 |
| composition CD(invention) | **45,4** | 2,4 |

Ces résultats montrent que l'utilisation du polymère substantif amphotère (Polyquaternium-22) seul (composition BD) ne permet pas de limiter la dégradation de la fibre par rapport à la composition contenant ni polymère substantif ni polymère amphiphile (composition AD).

En revanche, l'association selon l'invention d'un copolymère amphiphile anionique d'acide acrylique et d'acrylate d'alkyle en $C_{10-30}$ (CARBOPOL 1382 de la société GOODRICH) au polymère substantif amphotère Polyquaternium-22 (composition CD) réduit significativement, d'environ 17 %, la solubilité alcaline des fibres kératiniques décolorées.

**Exemple 4**

On a préparé la composition de décoloration sous forme pulvérulente, E, suivante (quantités en % en poids) :

| | |
|---|---|
| Persulfate de potassium | 38 |
| Persulfate de sodium | 30 |

(suite)

| | |
|---|---|
| Métasilicate de sodium | 14 |
| Chlorure d'ammonium | 5 |
| Acide éthylènediamine tétraacétique | 1 |
| Dioctylsulfosuccinate de Na/benzoate de Na | 2 |
| Silice | 2 |
| Polyquaternium- 22 | 1 |
| Cétylhydroxyéthylcellulose vendue sous la dénomination Polysurf 67 par la société HERCULES | 4 |

8g de cette composition E ont été mélangés à 16 g de la composition D décrite précédemment. On a appliqué et maintenu ce mélange sur des cheveux à décolorer pendant 45 minutes. On a obtenu une décoloration uniforme après rinçage, shampooing et séchage. L'état de la fibre était satisfaisant avec une dégradation limitée.

**Exemple 5**

On a préparé la composition de décoloration sous forme de crème anhydre, F, suivante (quantités en % en poids) :

| | |
|---|---|
| Palmitate d'isopropyle | 23 |
| Huile minérale | 3 |
| Persulfate de potassium | 27 |
| Persulfate de sodium | 20 |
| Métasilicate de sodium | 12 |
| Chlorure d'ammonium | 4 |
| Acide éthylène diamine tétraacétique | 1 |
| Alcool cétylstéarylique à 25 moles d'oxyde d'éthylène | 2 |
| Argile | 1 |
| Polyquaternium-22 | 1 |
| Serad FX-1100 | 3 |
| Oxyde de titane | 1 |
| Stéarate de magnésium | 2 |

10g de cette composition F ont été mélangés avec 15 g de la composition D décrite précédemment. On a appliqué et maintenu ce mélange sur des cheveux à décolorer pendant 45 minutes. On a obtenu une décoloration uniforme après rinçage, shampooing et séchage. L'état de la fibre était satisfaisant avec une dégradation limitée.

**Revendications**

1. Composition anhydre pour la décoloration de fibres kératiniques, en particulier de fibres kératiniques humaines, comprenant, dans un milieu approprié pour la décoloration, au moins un agent alcalin et au moins un sel peroxygéné, **caractérisée par le fait qu'**elle contient en outre l'association

   - d'au moins un polymère amphiphile anionique et/ou non ionique comportant au moins une chaîne grasse, et
   - d'au moins un polymère substantif cationique ou amphotère.

2. Composition selon la revendication 1, **caractérisée par le fait que** le polymère amphiphile anionique comportant au moins une chaîne grasse est un copolymère comprenant

   - des motifs hydrophiles dérivés d'un ou de plusieurs monomères à insaturation éthylénique portant une fonction d'acide carboxylique, et
   - des motifs hydrophobes dérivés d'un ou de plusieurs monomères à insaturation éthylénique portant une chaîne latérale hydrophobe.

3. Composition selon la revendication 1 ou 2 **caractérisée par le fait que** le ou les monomères à insaturation éthylénique portant une fonction d'acide carboxylique sont choisis parmi l'acide éthacrylique, l'acide méthacrylique et l'acide acrylique, de préférence parmi l'acide méthacrylique, l'acide acrylique et des mélanges de ceux-ci.

4. Composition selon l'une quelconque des revendications 1 à 3, **caractérisé par le fait que** le ou les monomères à insaturation éthylénique portant une chaîne latérale hydrophobe sont choisis parmi les éthacrylates, méthacrylates et/ou acrylates d'alkyle en $C_{10-30}$, de préférence en $C_{12-22}$.

5. Composition selon l'une quelconque des revendications 1 à 3, **caractérisée par le fait que** le ou les monomères à insaturation éthylénique portant une chaîne latérale hydrophobe sont choisis parmi les éthers allyliques d'alcools gras correspondant à la formule

(I) $CH_2=CR'CH_2-O-B_n-R$

dans laquelle

R' représente un atome d'hydrogène ou un groupe méthyle,
B représente un groupe éthylèneoxy,
n est un nombre entier valant entre 0 et 100,
R représente un groupe hydrocarboné choisi parmi les restes alkyle, arylalkyle, aryle, alkylaryle ou cycloalkyle comportant de 8 à 30 atomes de carbone, de préférence de 10 à 24 atomes de carbone.

6. Composition selon l'une quelconque des revendications précédentes, **caractérisée par le fait que** le polymère amphiphile anionique comportant au moins une chaîne grasse comprend en outre des motifs dérivés d'un monomère réticulant contenant deux doubles liaisons éthyléniques non conjuguées.

7. Composition selon la revendication 6, **caractérisée par le fait que** ledit monomère réticulant est choisi parmi le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.

8. Composition selon l'une quelconque des revendications 1 à 7, **caractérisée par le fait que** le polymère amphiphile anionique comportant au moins une chaîne grasse est un copolymère réticulé d'acide acrylique et d'acrylate d'alkyle en $C_{10-30}$.

9. Composition selon la revendication 1, **caractérisée par le fait que** ledit polymère amphiphile non ionique comportant au moins une chaîne grasse est choisi parmi

- les celluloses ou hydroxyalkylcelluloses modifiées par des groupements comportant au moins une chaîne grasse de type alkyle, arylalkyle ou alkylaryle contenant un groupe alkyle en $C_{8-22}$, ou par des groupes alkylphénol polyalcoxylés ;
- les hydroxypropylguars modifiés par des groupements comportant au moins une chaîne grasse en $C_{8-22}$ ;
- les polyuréthannes comportant au moins une chaîne grasse de type alkyle ou alcényle en $C_{8-30}$;
- les copolymères de vinylpyrrolidone et de monomères hydrophobes à chaîne grasse,
- les copolymères de (méth)acrylates d'alkyle en $C_{1-6}$ et de monomères amphiphiles comportant au moins une chaîne grasse,
- les copolymères de (méth)acrylates hydrophiles et de monomères hydrophobes comportant au moins une chaîne grasse.

10. Composition selon la revendication 9, **caractérisée par le fait que** le polymère amphiphile non ionique comportant au moins une chaîne grasse est une hydroxyéthylcellulose modifiée par des groupements comportant au moins un groupe alkyle en $C_{8-22}$ ou un polyuréthanne modifié par au moins une chaîne alkyle en $C_{10-20}$.

11. Composition selon l'une quelconque des revendications précédentes, **caractérisée par le fait que** le polymère amphiphile anionique et/ou le polymère amphiphile non ionique comportant au moins une chaîne grasse est (sont) présent(s) à raison de 0,03 à 30 % en poids, de préférence à raison de 0,3 à 15 % en poids par rapport à la composition anhydre totale.

**12.** Composition selon l'une quelconque des revendications précédentes, **caractérisée par le fait que** le polymère substantif cationique ou amphotère est un polymère contenant des motifs comportant des groupements amine primaire, secondaire, tertiaire et/ou quaternaire pouvant soit faire partie de la chaîne principale du polymère soit être portés par un substituant latéral directement relié à celle-ci, ayant une masse moléculaire moyenne en nombre comprise entre 500 et $5.10^6$, de préférence entre $10^3$ et $3 . 10^6$.

**13.** Composition selon la revendication 12, **caractérisée par le fait que** ledit polymère substantif est choisi parmi les dérivés de cellulose quaternisée et les polyacrylates à groupements latéraux aminés, quaternisés ou non.

**14.** Composition selon la revendication 13, **caractérisée par le fait que** les dérivés de cellulose quaternisée sont choisis parmi

- les celluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci, et
- les hydroxyéthylcelluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci.

**15.** Composition selon la revendication 12, **caractérisée par le fait que** ledit polymère substantif cationique est un homopolymère de chlorure de diméthyldiallylammonium ou un copolymère de diméthyldiallylammonium et d'acrylamide.

**16.** Composition selon la revendication 12, **caractérisée par le fait que** ledit polymère substantif cationique est un poly(ammonium quaternaire) constitué de motifs récurrents répondant à la formule

(XXI) 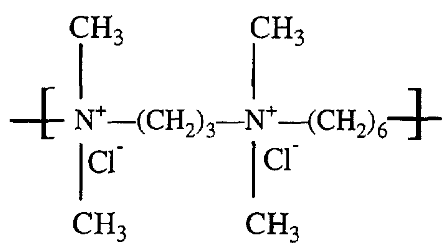

ou à la formule

(XXII) 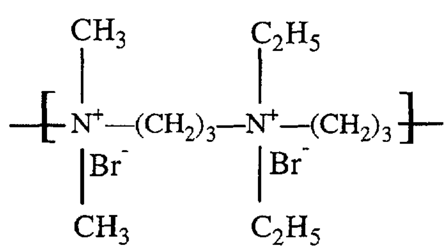

ou encore à la formule

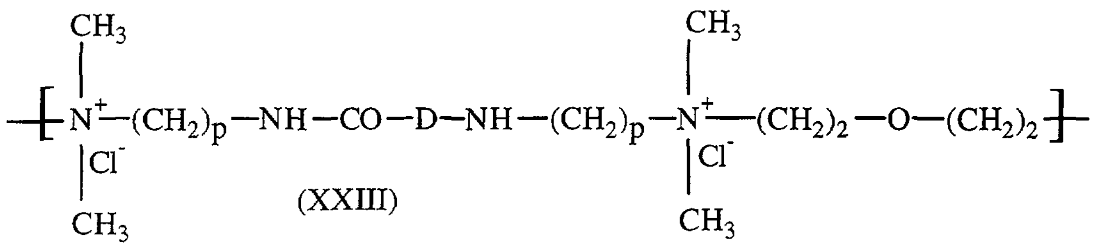

(XXIII)

dans laquelle

p est un nombre entier valant entre 1 et 6,
D représente une simple liaison ou un groupe $-(CH_2)_r-CO-$ où r vaut 4 ou 7.

**17.** Composition selon la revendication 12, **caractérisée par le fait que** ledit polymère substantif amphotère est choisi parmi le Polyquaternium-22, le Polyquaternium-39 et le Polyquaternium-47.

**18.** Composition selon l'une quelconque des revendications précédentes, **caractérisée par le fait que** ledit polymère substantif cationique ou amphotère est présent à raison de 0,03 à 30 % en poids, de préférence à raison de 0,3 à 15 % en poids par rapport à la composition anhydre totale.

**19.** Composition selon l'une quelconque des revendications précédentes, **caractérisée par le fait que** le rapport en poids du polymère amphiphile anionique et/ou non ionique au polymère substantif cationique ou amphotère est compris entre 10/1 et 1/10, de préférence entre 5/1 et 1/5.

**20.** Composition selon l'une quelconque des revendications précédentes, **caractérisée par le fait que** ledit agent alcalin est choisi parmi les silicates, phosphates ou carbonates de métaux alcalins ou alcalino-terreux, en particulier les métasilicates de métaux alcalins.

**21.** Composition selon l'une quelconque des revendications précédentes, **caractérisée par le fait que** ledit sel peroxygéné est choisi parmi les persulfates, percarbonates et perborates d'ammonium ou de métaux alcalins, en particulier parmi les persulfates de sodium et de potassium.

**22.** Composition selon la revendication 21, **caractérisée par le fait que** ledit sel peroxygéné est présent en une quantité allant de 20 à 70 %, et de préférence de 30 à 60 % en poids calculée par rapport à la composition anhydre.

**23.** Composition selon l'une quelconque des revendications précédentes, **caractérisée par le fait qu'**elle contient en outre des adjuvants de décoloration choisis parmi les agents de contrôle du dégagement d'oxygène, les polymères épaississants ou gélifiants, les agents tensioactifs anioniques, non ioniques, cationiques, amphotères ou zwitterioniques et leurs mélanges, les huiles minérales et végétales, les cires, les adjuvants de granulation, les liants, les charges minérales, les opacifiants tels que l'oxyde de titane, les colorants, les séquestrants et les parfums.

**24.** Composition selon la revendication 23, **caractérisée par le fait qu'**elle contient de 0,01 à 40 % en poids, de préférence de 0,1 à 30 % en poids d'au moins un agent tensioactif.

**25.** Composition selon l'une quelconque des revendications précédentes, **caractérisée en ce qu'**elle se présente sous forme d'une poudre, ou d'une suspension ou dispersion de poudre dans un support liquide organique anhydre.

**26.** Utilisation de la composition anhydre décolorante selon l'une quelconque des revendications précédentes pour la préparation d'une composition décolorante prête-à-l'emploi par addition d'une composition aqueuse de peroxyde d'hydrogène.

**27.** Procédé de décoloration de fibres kératiniques, en particulier de cheveux humains, comprenant les étapes consistant

- à mélanger, immédiatement avant emploi, une composition décolorante anhydre définie selon l'une quelconque des revendications 1 à 25, avec une composition aqueuse de peroxyde d'hydrogène,
- à appliquer le mélange obtenu sur la zone des fibres kératiniques à décolorer,
- à laisser reposer pendant un temps suffisant pour obtenir la décoloration recherchée, et
- à éliminer le mélange décolorant par rinçage à l'eau suivi d'un lavage avec un shampooing, puis d'un séchage.

**28.** Dispositif à plusieurs compartiments, ou "kit", pour la décoloration des fibres kératiniques et en particulier des fibres kératiniques humaines telles que les cheveux, **caractérisé par le fait qu'**il comporte au moins deux compartiments dont l'un contient une composition anhydre selon l'une quelconque des revendications 1 à 25, et l'autre une composition aqueuse de peroxyde d'hydrogène.

## Patentansprüche

**1.** Wasserfreie Zusammensetzung zum Bleichen von Keratinfasern und insbesondere menschlichen Keratinfasern, die in einem zum Bleichen geeigneten Medium mindestens eine Base und mindestens ein Peroxidsalz enthält, **dadurch gekennzeichnet, daß** sie ferner die folgende Kombination enthält:

- mindestens ein anionisches und/oder nichtionisches amphiphiles Polymer, das mindestens eine Fettkette aufweist-, und
- mindestens ein kationisches oder amphoteres substantives Polymer.

**2.** Zusammensetzung nach Anspruch 1, **dadurch gekennzeichnet, daß** das anionische amphiphile Polymer, das mindestens eine Fettkette aufweist, ein Copolymer ist, das enthält:

- *hydrophile* Einheiten, die von einem oder mehreren Monomeren mit ethylenischer Doppelbindung abgeleitet sind, die eine Carbonsäuregruppe aufweisen, und
- *hydrophobe* Einheiten, die von einem oder mehreren Monomeren mit ethylenischer Doppelbindung abgeleitet sind, die eine hydrophobe Seitenkette aufweisen.

**3.** Zusammensetzung nach Anspruch 1 oder 2, **dadurch gekennzeichnet, daß** das Monomer oder die Monomere mit ethylenischer Doppelbindung, die eine Carboxygruppe tragen, unter Ethacrylsäure, Methacrylsäure und Acrylsäure und vorzugsweise Methacrylsäure und Acrylsäure und ihren Gemischen ausgewählt sind.

**4.** Zusammensetzung nach einem der Ansprüche 1 bis 3, **dadurch gekennzeichnet, daß** das Monomer oder die Monomere mit ethylenischer Doppelbindung, die eine hydrophobe Seitenkette aufweisen, unter den Ethacrylaten, Methacrylaten und/oder Acrylaten von $C_{10-30}$-Alkyl und vorzugsweise $C_{12-22}$-Alkyl ausgewählt sind.

**5.** Zusammensetzung nach einem der Ansprüche 1 bis 3, **dadurch gekennzeichnet, daß** das Monomer oder die Monomere mit ethylenischer Doppelbindung, die eine hydrophobe Seitenkette aufweisen, unter den Allylethern von Fettalkoholen der folgenden Formel ausgewählt sind:

$$(I) \qquad CH_2=CR'CH_2-O-B_n-R$$

worin bedeuten:

R' ein Wasserstoffatom oder die Methylgruppe,
B Ethylenoxy,
n Null oder eine ganze Zahl von 1 bis 100,
R eine Kohlenwasserstoffgruppe, die unter Alkyl, Arylalkyl, Aryl, Alkylaryl oder Cycloalkyl mit 8 bis 30 Kohlenstoffatomen und vorzugsweise 10 bis 24 Kohlenstoffatomen ausgewählt ist.

**6.** Zusammensetzung nach einem der vorhergehenden Ansprüche, **dadurch gekennzeichnet, daß** das anionische amphiphile Polymer, das mindestens eine Fettkette aufweist, ferner Einheiten enthält, die von einem vernetzenden Monomer abgeleitet sind, das nicht konjugierte ethylenische Doppelbindungen enthält.

**7.** Zusammensetzung nach Anspruch 6, **dadurch gekennzeichnet, daß** das vernetzende Monomer unter Diallylphthalat, Allyl(meth)acrylat, Divinylbenzol, (Poly)ethylenglykoldimethacrylat, Methylen-bisacrylamid, Polyallysaccharose und Polyallylpentaerythrit ausgewählt ist.

**8.** Zusammensetzung nach einem der Ansprüche 1 bis 7, **dadurch gekennzeichnet, daß** das anionische amphiphile Polymer, das mindestens eine Fettkette aufweist, ein vernetztes Copolymer von Acrylsäure und $C_{10-30}$-Alkylacrylat ist.

**9.** Zusammensetzung nach Anspruch 1, **dadurch gekennzeichnet, daß** das nichtionische amphiphile Polymer, das mindestens eine Fettkette aufweist, ausgewählt ist unter:

- Cellulosen oder Hydroxyalkylcellulosen, die mit Gruppen, die mindestens eine Fettkette vom Typ Alkyl, Arylalkyl oder Alkylaryl aufweisen, die vorzugsweise eine $C_{8-22}$-Alkylgruppe enthalten, oder mit polyalkoxylierten Alkylphenolgruppen modifiziert sind;
- Hydroxypropylguarverbindungen, die mit Gruppen modifiziert sind, die mindestens eine $C_{8-22}$-Fettkette aufweisen;
- Polyurethanen, die mindestens eine Fettkette vom Typ Alkyl oder Alkenyl mit 8 bis 30 Kohlenstoffatomen aufweisen;
- Copolymeren von Vinylpyrrolidon und hydrophoben Monomeren mit Fettkette;
- Copolymeren von $C_{1-6}$-Alkyl(meth)acrylaten und amphiphilen Monomeren, die mindestens eine Fettkette aufweisen; und
- Copolymeren von hydrophilen (Meth)acrylaten und hydrophoben Monomeren, die mindestens eine Fettkette aufweisen.

**10.** Zusammensetzung nach Anspruch 9, **dadurch gekennzeichnet, daß** es sich bei dem nichtionischen amphiphilen Polymer, das mindestens eine Fettkette aufweist, um eine Hydroxyethylcellulose, die mit Gruppen modifiziert sind, die mindestens eine $C_{8-22}$-Alkylgruppe enthalten, oder um ein Polyurethan handelt, das mit mindestens einer $C_{10-20}$-Alkylgruppe modifiziert ist.

**11.** Zusammensetzung nach einem der vorhergehenden Ansprüche, **dadurch gekennzeichnet, daß** das anionische amphiphile Polymer und/oder das nichtionische amphiphile Polymer, die mindestens eine Fettkette aufweisen, in einem Mengenanteil von 0,03 bis 30 Gew.-% und vorzugsweise 0,3 bis 15 Gew.-%, bezogen auf die gesamte wasserfreie Zusammensetzung, vorliegt (vorliegen).

**12.** Zusammensetzung nach einem der vorhergehenden Ansprüche, **dadurch gekennzeichnet, daß** das kationische oder amphotere substantive Polymer ein Polymer ist, das entweder als Teil der Polymerhauptkette oder an einem direkt an die Hauptkette gebundenen seitlichen Substituenten Einheiten mit primären, sekundären, tertiären und/oder quartären Aminogruppen/Ammoniumgruppen enthält und eine Zahlenmittel des Molekulargewichts von 500 bis $5 \cdot 10^6$ und vorzugsweise $10^3$ bis $3 \cdot 10^6$ aufweist.

**13.** Zusammensetzung nach Anspruch 12, **dadurch gekennzeichnet, daß** das substantive Polymer unter den quaternisierten Cellulosederivaten und Polyacrylaten mit aminierten, gegebenenfalls quaternisierten Seitengruppen ausgewählt ist.

**14.** Zusammensetzung nach Anspruch 13, **dadurch gekennzeichnet, daß** die quaternisierten Cellulosederivate ausgewählt sind unter:

- quaternisierten Cellulosen, die mit Gruppen modifiziert sind, die mindestens eine Fettkette aufweisen und unter Alkyl-, Arylalkyl- oder Alkylarylgruppen mit mindestens 8 Kohlenstoffatomen ausgewählt sind, oder deren Gemischen;
- quaternisierten Hydroxyethylcellulosen, die mit Gruppen modifiziert sind, die mindestens eine Fettkette aufweisen und unter Alkyl-, Arylalkyl- oder Alkylarylgruppen mit mindestens 8 Kohlenstoffatomen ausgewählt sind, oder deren Gemischen.

**15.** Zusammensetzung nach Anspruch 12, **dadurch gekennzeichnet, daß** das kationische substantive Polymer ein Homopolymer von Dimethyldiallylammoniumchlorid oder ein Copolymer von Dimethyldiallylammoniumchlorid und Acrylamid ist.

**16.** Zusammensetzung nach Anspruch 12, **dadurch gekennzeichnet, daß** das kationische substantive Polymer ein Poly(quartäres Ammonium)-Polymer ist, das aus wiederkehrenden Einheiten der Formel

(XXI) 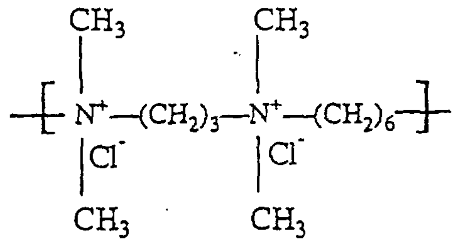

oder der Formel

(XXII) 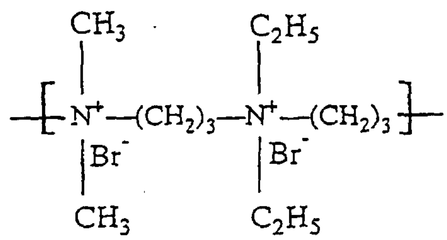

oder der Formel

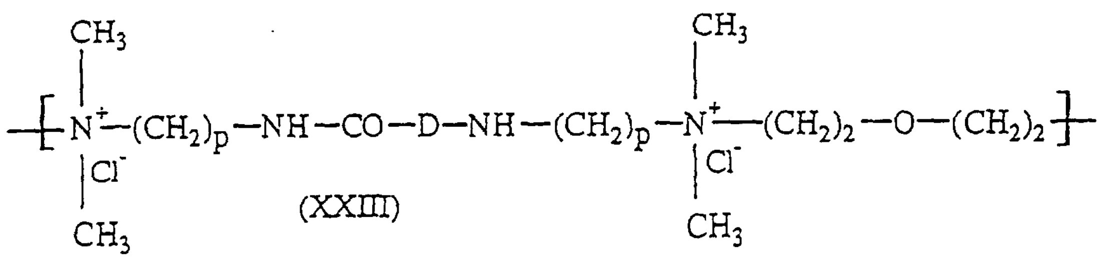
(XXIII)

besteht, worin bedeuten:

p eine ganze Zahl von 1 bis 6, und
D eine Einfachbindung oder eine Gruppe $-(CH_2)_r-CO-$ mit r = 4 oder 7.

**17.** Zusammensetzung nach Anspruch 12, **dadurch gekennzeichnet, daß** das amphotere substantive Polymer unter Polyquaternium-22, Polyquaternium-39 und Polyquaternium-47 ausgewählt ist.

**18.** Zusammensetzung nach einem der vorhergehenden Ansprüche, **dadurch gekennzeichnet, daß** das kationische oder amphotere substantive Polymer in einem Mengenanteil von 0,03 bis 30 Gew.-% und vorzugsweise 0,3 bis 15 Gew.-%, bezogen auf die gesamte wasserfreie Zusammensetzung, vorliegt.

**19.** Zusammensetzung nach einem der vorhergehenden Ansprüche, **dadurch gekennzeichnet, daß** das Gewichtsverhältnis des anionischen und/oder nichtionischen amphiphilen Polymers und des kationischen oder amphoteren substantiven Polymers im Bereich von 10/1 bis 1/10 und vorzugsweise 5/1 bis 1/5 liegt.

**20.** Zusammensetzung nach einem der vorhergehenden Ansprüche, **dadurch gekennzeichnet, daß** die Base unter den Alkalisilicaten, Alkaliphosphaten, Alkalicarbonaten, Erdalkalisilicaten, Erdalkaliphosphaten oder Erdalkalicarbonaten und insbesondere Alkalimetasilicaten ausgewählt ist.

21. Zusammensetzung nach einem der vorhergehenden Ansprüche, **dadurch gekennzeichnet, daß** das Peroxidsalz unter den Persulfaten, Percarbonaten und Perboraten von Ammonium oder Alkalimetallen und insbesondere Natriumpersulfat und Kaliumpersulfat ausgewählt ist.

22. Zusammensetzung nach Anspruch 21, **dadurch gekennzeichnet, daß** das Peroxidsalz in einer Menge von 20 bis 70 Gew.-% und vorzugsweise 30 bis 60 Gew.-%, bezogen auf die wasserfreie Zusammensetzung, vorliegt.

23. Zusammensetzung nach einem der vorhergehenden Ansprüche, **dadurch gekennzeichnet, daß** sie ferner Zusatzstoffe zum Bleichen enthält, die unter Mitteln zur Regulierung der Sauerstoffentwicklung, verdickenden Polymeren oder Gelbildnern, anionischen, nichtionischen, kationischen, amphoteren oder zwitterionischen grenzflächenaktiven Stoffen und ihren Gemischen, Mineralölen, pflanzlichen Ölen, Wachsen, Hilfsstoffen für die Granulierung, Bindemitteln, anorganischen Füllstoffen, Trübungsmitteln, wie Titanoxid, Färbemitteln, Maskierungsmitteln und Parfums ausgewählt sind.

24. Zusammensetzung nach Anspruch 23, **dadurch gekennzeichnet, daß** sie 0,01 bis 40 Gew.-% und vorzugsweise 0,1 bis 30 Gew.-% mindestens eines grenzflächenaktiven Stoffes enthält.

25. Zusammensetzung nach einem der vorhergehenden Ansprüche, **dadurch gekennzeichnet, daß** sie in Form eines Pulvers oder einer Suspension oder Dispersion von Pulver in einem wasserfreien flüssigen organischen Träger vorliegt.

26. Verwendung der wasserfreien Zusammensetzung zum Bleichen nach einem der vorhergehenden Ansprüche zur Herstellung einer gebrauchsfertigen Zusammensetzung zum Bleichen durch Zusatz einer wäßrigen Wasserstoffperoxid-Zubereitung.

27. Verfahren zum Bleichen von Keratinfasern und insbesondere menschlichen Haaren mit folgenden Schritten:

- die wasserfreie Zusammensetzung zum Bleichen nach einem der Ansprüche 1 bis 25 wird unmittelbar vor der Anwendung mit einer wäßrigen Wasserstoffperoxid-Zubereitung vermischt,
- das Gemisch wird auf den Bereich der zu blondierenden Keratinfasern aufgetragen,
- das Gemisch wird während einer Zeitspanne, die ausreichend ist, um die gewünschte Blondierung zu erzielen, einwirken gelassen, und
- das Gemisch zum Bleichen wird durch Spülen mit Wasser entfernt, worauf mit Haarwaschmittel gewaschen und getrocknet wird.

28. Vorrichtung mit mehreren Abteilungen oder "Kit" zum Bleichen von Keratinfasern und insbesondere menschlichen Haaren, wie dem Haar, **dadurch gekennzeichnet, daß** sie mindestens zwei Abteilungen aufweist, wobei eine Abteilung eine wasserfreie Zusammensetzung nach einem der Ansprüche 1 bis 25 und eine andere Abteilung eine wäßrige Wasserstoffperoxid-Zubereitung enthält.

## Claims

1. Anhydrous composition for bleaching keratin fibres, in particular human keratin fibres, comprising, in a medium which is suitable for bleaching, at least one alkaline agent and at least one peroxygenated salt, **characterized in that** it contains, in addition, a combination

- of at least one anionic and/or nonionic amphiphilic polymer comprising at least one fatty chain, and
- of at least one cationic or amphoteric substantive polymer.

2. Composition according to Claim 1, **characterized in that** the anionic amphiphilic polymer comprising at least one fatty chain is a copolymer comprising

- hydrophilic units derived from one or more monomers containing ethylenic unsaturation bearing a carboxylic acid function, and
- hydrophobic units derived from one or more monomers containing ethylenic unsaturation bearing a hydrophobic side chain.

**3.** Composition according to Claim 1 or 2, **characterized in that** the monomer(s) containing ethylenic unsaturation bearing a carboxylic acid function is(are) chosen from ethacrylic acid, methacrylic acid and acrylic acid, preferably from methacrylic acid and acrylic acid and mixtures thereof.

**4.** Composition according to any one of Claims 1 to 3, **characterized in that** the monomer(s) containing ethylenic unsaturation bearing a hydrophobic side chain is(are) chosen from $C_{10}$-$C_{30}$, preferably $C_{12}$-$C_{22}$, alkyl ethacrylates, methacrylates and/or acrylates.

**5.** Composition according to any one of Claims 1 to 3, **characterized in that** the monomer(s) containing ethylenic unsaturation bearing a hydrophobic side chain is(are) chosen from allyl fatty alkyl ethers corresponding to the formula

$$(I) \qquad CH_2{=}CR'CH_2\text{-}O\text{-}B_n\text{-}R$$

in which

R' represents a hydrogen atom or a methyl group,
B represents an ethylenoxy group,
n is an integer between 0 and 100,
R represents a hydrocarbon-based group chosen from alkyl, arylalkyl, aryl, alkylaryl and cycloalkyl residues comprising from 8 to 30 carbon atoms, preferably from 10 to 24 carbon atoms.

**6.** Composition according to any one of the preceding claims, **characterized in that** the anionic amphiphilic polymer comprising at least one fatty chain also comprises units derived from a crosslinkingmonomer containing two non-conjugated ethylenic double bonds.

**7.** Anhydrous composition according to Claim 6, **characterized in that** the said crosslinking monomer is chosen from diallyl phthalate, allyl (meth)acrylate, divinylbenzene, (poly)ethylene glycol dimethacrylate, methylenebisacrylamide, polyallylsucrose or polyallylpentaerythritol.

**8.** Composition according to any one of Claims 1 to 7, **characterized in that** the anionic amphiphilic polymer comprising at least one fatty chain is a crosslinked copolymer of acrylic acid and of $C_{10-30}$ alkyl acrylate.

**9.** Composition according to claim 1, **characterized in that** the said nonionic amphiphilic polymer comprising at least one fatty chain is chosen from

- celluloses or hydroxyalkylcelluloses modified with groups comprising at least one fatty chain of alkyl, arylalkyl or alkylaryl type containing a $C_8$-$C_{22}$ alkyl group, or with polyalkoxylated alkylphenol groups;
- hydroxypropyl guars modified with groups comprising at least one $C_8$-$C_{22}$ fatty chain;
- polyurethanes comprising at least one fatty chain of $C_8$-$C_{30}$ alkyl or alkenyl type;
- copolymers of vinylpyrrolidone and of hydrophobic monomers containing a fatty chain;
- copolymers of $C_1$-$C_6$ alkyl (meth)acrylates and of amphiphilic monomers comprising at least one fatty chain;
- copolymers of hydrophilic (meth)acrylates and of hydrophobic monomers comprising at least one fatty chain.

**10.** Composition according to Claim 9, **characterized in that** the nonionic amphiphilic polymer comprising at least one fatty chain is a hydroxyethylcellulose modified with groups comprising at least one $C_8$-$C_{22}$ alkyl group or a polyurethane modified with at least one $C_{10}$-$C_{20}$ alkyl chain.

**11.** Composition according to any one of the preceding claims, **characterized in that** the anionic amphiphilic polymer and/or the nonionic amphiphilic polymer comprising at least one fatty chain is(are) present in a proportion of from'0.03 to 30% by weight, preferably in a proportion of from 0.3 to 15% by weight, relative to the total anhydrous composition.

**12.** Composition according to any one of the preceding claims, **characterized in that** the cationic or amphoteric substantive polymer is a polymer containing units comprising primary, secondary, tertiary and/or quaternary amine groups which can either form part of the main. chain of the polymer or can be borne by a side substituent which is directly connected thereto, having a number-average molecular mass of between 500 and $5 \times 10^6$, preferably

between $10^3$ and $3\times10^6$.

**13.** Composition according to Claim 12, **characterized in that** the said substantive polymer is chosen from quaternized cellulose derivatives and polyacrylates containing quaternized or non-quaternized amino side groups.

**14.** Composition according to Claim 13, **characterized in that** the quaternized cellulose derivatives are chosen from

- quaternized celluloses modified with groups comprising at least one fatty chain, chosen from alkyl, arylalkyl and alkylaryl groups comprising at least 8 carbon atoms, or mixtures thereof, and
- quaternized hydroxyethylcelluloses modified with groups comprising at least one fatty chain, chosen from alkyl, arylalkyl and alkylaryl groups comprising. at least 8 carbon atoms, or mixtures thereof.

**15.** Composition according to Claim 12, **characterized in that** the said cationic substantive polymer is a dimethyldiallylammonium chloride homopolymer or a copolymer of dimethyldiallylammonium [lacuna] and of acrylamide.

**16.** Composition according to Claim 12, **characterized in that** the said cationic substantive polymer is a poly(quaternary ammonium) consisting of repeating units corresponding to the formula

(XXI) 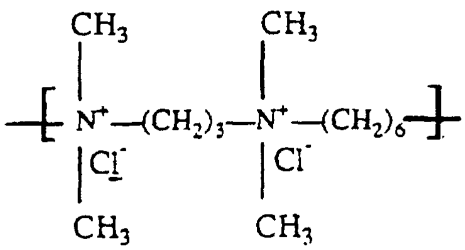

or to the formula

(XXII) 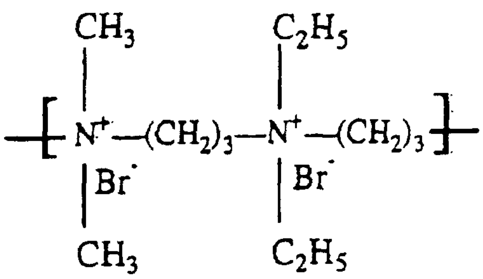

or alternatively to the formula

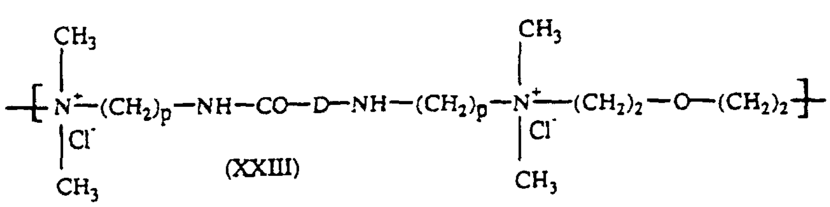

(XXIII)

in which

p is an integer between 1 and 6,
D represents a single bond or a group $-(CH_2)_r-CO-$ in which r is 4 or 7.

**17.** Composition according to Claim 12, **characterized in that** the said amphoteric substantive polymer is chosen from Polyquaternium-22, Polyquaternium-39 and Polyquaternium-47.

**18.** Composition according to any one of the proceding claims, **characterized in that** the said cationic or amphoteric substantive polymer is present in a proportion of from 0.03 to 30% by weight, preferably in a proportion of from 0.3 to 15% by weight, relative to the total anhydrous composition.

**19.** Composition according to any one of the preceding claims, **characterized in that** the weight ratio of the anionic and/or nonionic amphiphilic polymer to the cationic or amphoteric substantive polymer is between 10/1 and 1/10, preferably between 5/1 and 1/5.

**20.** Composition according to any one of the preceding claims, **characterized in that** the said alkaline agent is chosen from alkali metal or alkalineearth metal silicates, phosphates or carbonates, in particular alkali metal metasilicates.

**21.** Composition according to any one of the preceding claims, **characterized in that** the said peroxygenated salt is chosen from the ammonium and alkali metal persulphates, percarbonates and perborates, in particular from sodium persulphate and potassium persulphate.

**22.** Composition according to Claim 21, **characterized in that** the said peroxygenated salt is present in an amount ranging from 20 to 70%, and preferably from 30 to 60%, by weight calculated relative to the anhydrous composition.

**23.** Composition according to any one of the preceding claims, **characterized in that** it also contains bleaching adjuvants chosen from agents for controlling the release of oxygen, thickening or gelling polymers, anionic, nonionic, cationic, amphoteric or zwitterionic surfactants and mixtures thereof, mineral or plant oils, waxes, granulating adjuvants, binders, mineral fillers, opacifiers such as titanium oxide, dyes, sequestering agents and fragrances.

**24.** Composition according to Claim 23, **characterized in that** it contains from 0.01 to 40% by weight, preferably from 0.1 to 30% by weight, of at least one surfactant.

**25.** Composition according to any one of the preceding claims, **characterized in that** it is in the form of a powder, or of a suspension or dispersion of powder in an anhydrous organic liquid support.

**26.** Use of the anhydrous bleaching composition according to any one of the preceding claims to prepare a ready-to-use bleaching composition by addition of an aqueous hydrogen peroxide composition.

**27.** Process for bleaching keratin fibres, in particular human hair, comprising the steps consisting

- in mixing, immediately before use, an anhydrous bleaching composition defined according to any one of Claims 1 to 25 with an aqueous hydrogen peroxide composition,
- in applying the mixture obtained to the region of keratin fibres to be bleached,
- in leaving the mixture to stand on the fibres for a period which is sufficient to obtain the desired bleaching effect, and
- in removing the bleaching mixture by rinsing with water, followed by washing with a shampoo, and then drying.

**28.** Multi-compartment device or "kit" for bleaching keratin fibres, and in particular human keratin fibres such as the hair, **characterized in that** it comprises at least two compartments, one of which contains an anhydrous composition according to any one of Claims 1 to 25, and the other of which contains an aqueous hydrogen peroxide composition.